# EUROPEAN PATENT SPECIFICATION

(11) **EP 0 440 163 B1**
(45) Date of publication and mention of the grant of the patent: **05.04.1995**
(21) Application number: 91101138.5
(22) Date of filing: 29.01.1991
(51) Int. Cl.: A61F 13/58

(54) **Disposable diaper**
Wegwerfwindel
Couche à jeter

(30) Priority: 30.01.1990 JP 17762/90
(43) Date of publication of application: 07.08.1991
(73) Proprietor: OJI PAPER CO. LTD., Tokyo 163 (JP); NEPIA CO., LTD., Chuo-ku, Tokyo 104 (JP)
(72) Inventor: Terada, Sadayoshi, Koto-ku, Tokyo (JP); Tanaka, Hisashi, Koto-ku, Tokyo (JP)
(74) Representative: KUHNEN, WACKER & PARTNER

(56) References cited:
- FR-A- 2 617 682
- GB-A- 2 183 174
- GB-A- 2 185 176
- US-A- 4 753 649

## Description

The present invention relates to a disposable diaper and to a method for producing same. More particularly, the present invention relates to a disposable diaper provided with an improved fastening system in which the outside surface of a longitudinal end portion of the diaper is reinforced by reinforcing resinous layers to which fastening adhesive tape members are detachably adhered.

A conventional disposable diaper comprises a liquid-nonpermeable back sheet consisting of, for example, a polyethylene film, a liquid-permeable front sheet consisting of, for example, a polyester nonwoven fabric, a liquid absorptive stratum interposed between the back and front sheets and comprising a fluff pulp mat, absorbent paper and/or a high water-absorbing (super absorbent) polymer material, and elastic stretchable members arranged on portions of the diaper which are brought into contact with the waist and crotch of the wearer.

Also, the conventional disposable diaper is provided with a fastening tape system for connecting a back portion of the diaper to a front portion thereof.

The conventional fastening tape system comprises a pair of fastening adhesive tape members located at both of the lateral side edge (corner) portions of the back portion of the diaper and able to be detachably adhered to both of the lateral side edge (corner) portions of the front portion of the diaper.

In the conventional disposable diaper, the lateral side edge portions of the front portion are reinforced by a reinforcing plastic tape having a relatively large width of about 15 to 25 cm and fixed to the longitudinal end portion of the front portion of the diaper.

The use of the reinforcing plastic tape requires operations of cutting a reinforcing plastic tape material into desired dimensions, and bonding the cut plastic tape to the back portion surface of the diaper, and thus leads to an increase in the cost and a reduction in the productivity of the diaper.

GB-A-2 185 176 discloses a diaper according to the preamble of claim 1. There, one or more strips of a reinforcing fastening tape are affixed to the diaper in such a position that the strips of adhesive fastening tape can be releasably adhered thereto when closing the diaper.

GB-A-2 183 174 is directed to a breathable backing or release liner and a process for forming the same. The breathable backing or release liner has a pressalbe, woven or non-woven support member on one side of which a silicone-release coating is applied. The coating is formed by applying a radiation-curable composition containing a polysiloxane on one side of the support and exposing the composition to radiation so that the composition does not substantially penetrate the support.

An object of the present invention is to provide a disposable diaper having an improved fastening tape system by which the back portion of the diaper can be detachably and repeatedly fastened to the front portion thereof.

Another object of the present invention is to provide a disposable diaper provided with an improved fastening tape system having reinforcing resinous layers to which fastening tapes are detachably adhered, and which are easily formed on the outside surface of a longitudinal end portion of the diaper.

The above-mentioned objects can be attained by the disposable diaper according to claim 1. Furthermore, the invention provides a method according to claim 6.
Figure 1 is a back side plane view of an embodiment of the disposable diaper of the present invention;
Fig. 2 is a cross-sectional view of the disposable diaper shown in Fig. 1, taken along the line X₁ - X₂ of Fig. 1;
Fig. 3 is a back side plane view of another embodiment of the disposable diaper of the present invention; and,
Fig. 4 is a flow sheet showing a process for producing the disposable diaper of the present invention.

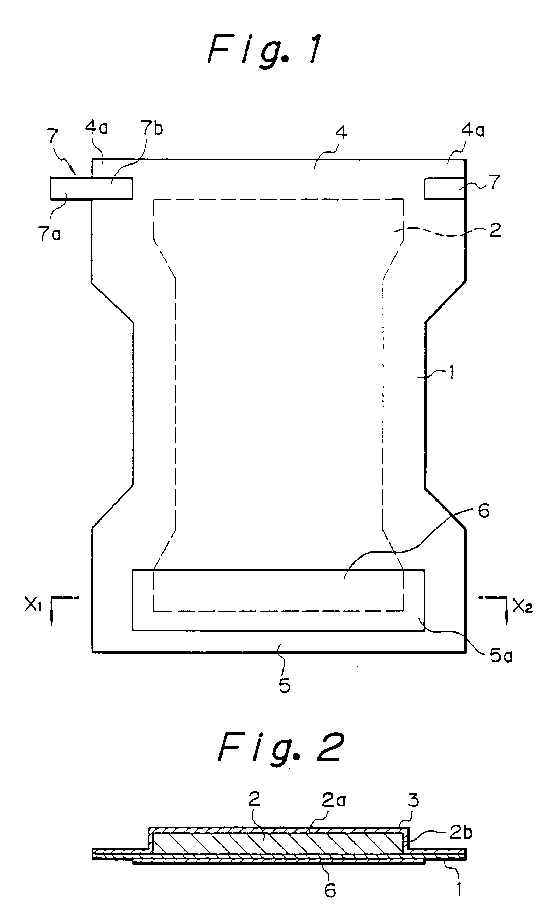

Referring to Figures 1, 2 and 3, the disposable diaper of the present invention comprises a liquid-nonpermeable back sheet 1, a liquid-absorptive core stratum 2 superimposed on a lateral middle portion of the back sheet 1, and a liquid-permeable front sheet 3 covering at least the upper surface 2a and both of the lateral side surfaces 2b of the liquid-absorptive core stratum.

The back surface of the diaper is formed by the liquid-nonpermeable back sheet.

The disposable diaper of the present invention is provided with an improved fastening tape system for detachably fastening a back portion 4 of the diaper to a front portion 5 thereof. This fastening tape system comprises at least one reinforcing resinous layer 6 coated on at least corner portions 5a of the back surface of a longitudinal end portion, preferably the front portion 5 of the diaper, and a pair of fastening tape members 7 each having an adhesive tape member 7a extending outward from a corner portion 4a of the back surface of the longitudinal opposite end portion, preferably the back portion 4 of the diaper. The adhesive tape member 7a has an adhesive layer, formed on the inside surface thereof, able to be detachably adhered to the corresponding portion of the reinforcing resinous layer at a peeling strength of 250 to 1000 g/25 mm, as determined in accordance with Japanese Industrial Standard (JIS) Z 0237, at a peeling speed of 200 mm/minute, at a peeling angle of 180 degrees, and at a width of a specimen to be tested of 25 mm.

If the peeling strength is less than 250 g/25 mm, the back portion of the diaper cannot be firmly fastened to the front portion thereof, and if the peeling strength is more than 1000 g/25 mm, it is difficult to detach the back portion of the diaper from the front portion thereof.

In the improved fastening tape system of the present invention, the reinforcing resinous layer comprises a product of a synthetic polymeric material cured by an irradiation of actinic rays, i.e., ultraviolet rays or an electron beam.

The reinforcing resinous layer of the present invention can be formed by coating a coating material containing a synthetic prepolymeric material able to be cured upon an irradiation of ultraviolet rays or an electron beam, a cross-linking or diluting monomeric agent, a polymerization-initiating agent, and optionally, a polymerization-inhibiting agent, on at least corner portions of the back surface of a longitudinal end portion, preferably the front portion, of the diaper, and irradiating the ultraviolet rays or the electron beam onto the coated layer of the coating material, to thereby cure or harden the coated layer.

The ultraviolet ray or electron beam-curable prepolymeric material usable for the present invention is not limited to a specific type of compound, and can be any compound which can be cured by an irradiation thereon of ultraviolet rays or an electron beam; the resultant cured resinous layer having no or only a very small barrier effect against an oxygen-permeation therethrough, exhibits an adequate softness and flexibility, can be rapidly cured by the irradiation of the ultraviolet rays or an electron beam, and can be adhered to the fastening tape member at the above-mentioned specific peeling strength.

Preferably, the ultraviolet ray or electron beam-curable prepolymeric material comprises at least one member selected from the group consisting of:
(1) acrylate compounds of aliphatic, cycloaliphatic and aromatic mono- to hexa-hydric alcohols and of polyalkylene glycols,
(2) acrylate compounds consisting of addition reaction products of aliphatic, cycloaliphatic and aromatic mono- to hexa-hydric alcohols with alkylene oxides,
(3) polyacryloylalkyl phosphoric acid esters,
(4) reaction products of carboxylic acids with polyols and acrylic acid,
(5) reaction products of isocyanate compounds with polyols and acrylic acid,
(6) reaction products of epoxy group-containing compounds with acrylic acid, and
(7) reaction products of epoxy group-containing compounds with polyols and acrylic acid.

More preferably, the prepolymeric material comprises at least one member selected from the group consisting of polyoxyethyleneepichlorohydrin-modified bisphenol A diacrylates, dicyclohexylacrylate, epichlorohydrin-modified polyethyleneglycol diacrylates, 1,6-hexamediol di-acrylate, hydroxypivalic acid ester-neopentyl glycol diacrylate, nonylphenoxypolyethyleneglycol acrylate, ethylene oxide-modified phenoxylated phosphoric acid acrylates, ethylene oxide-modified phthalic acid acrylates, polybutadiene acrylate, caprolactam-modified tetrahydrofurfuryl acrylates, tris(acryloxyethyl)isocyanulate, trimethylolpropane triacrylate, pentaerythritol triacrylate, dipentaerythritol hexaacrylate, polyethyleneglycol diacrylate, 1,4-butadienediol diacrylate, neopentylglycol diacrylate, and neopentylglycol-modified trimethylolpropane diacrylate.

The cross-linking or diluting monomeric agent preferably comprises at least one member selected from the group consisting of low molecular polyol acrylates, addition reaction products of polyether compounds and of polyol compounds with alkylene oxides, polybasic carboxylic acid-modified polyesters and polyols.

Also, the polymerization-initiating agent preferably comprises at least one member usable for conventional ultraviolet ray or electron beam polymerizations and selected from, for example, the group consisting of polymerization-initiating ketone and aminoketone compounds containing at least one member selected from benzoic structure, acetophenone structure and benzoinketol structure.

There is no restriction on the form and area of the reinforcing resinous layer as long as the layer reinforces at least the corner portions of the longitudinal end portion, preferably the front portion, of the diaper and firmly fastens the front portion of the diaper to the back portion thereof.

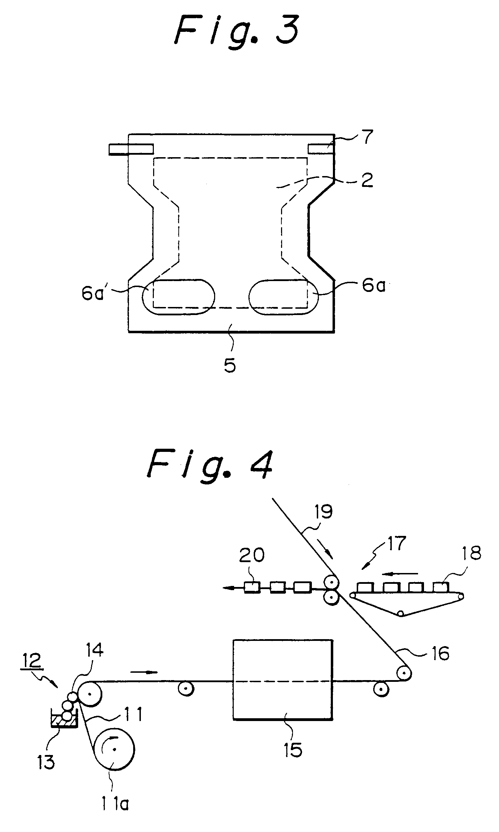

For example, the reinforcing resinous layer can be in the form of an oblong, rectangle, circle or oval. In an embodiment of the diaper of the present invention as indicated in Fig. 1, the reinforcing resinous layer 6 extends to one corner portion from the opposite corner portion of the back surface of the longitudinal end portion, preferably the front portion, of the diaper. In another embodiment of the diaper of the present invention as shown in Fig. 3, the reinforcing resinous layer is composed of a pair of reinforcing resinous layer segments 6a spaced from each other and located on corner portions of the back surface of the longitudinal end portion, preferably the front portion 5, of the diaper. These reinforcing resinous layer segments 6a can be in the form of an oblong, rectangle, circle or oval.

The reinforcing resinous layer of the present invention can be formed by a flexo printing method or a gravure printing method. Also, the reinforcing resinous layer can be continuously formed by using, for example, the printing or coating apparatus as shown in Fig. 4.

Referring to Fig. 4, a liquid-nonpermeable sheet 11, for example, a polyethylene film, is withdrawn from a roll 11a thereof and fed into a printing section 12, in which a coating resinous liquid 13 is applied in a predetermined pattern and area to a back surface of the sheet 11 through printing rolls 14. The printed layer of the coating resinous liquid 13 is cured in a curing section 15 in which ultraviolet rays or an electron beam are irradiated to the printed resinous liquid layer.

The resultant sheet 16 having the cured reinforcing resinous layer formed on the back surface thereof is fed into a diaper-forming section 17, and associated therein with a liquid-absorptive core material 18 and a liquid-permeable sheet material 19 to produce a diaper 20, in accordance with a usual disposable diaper-producing process.

For example, the liquid nonpermeable sheet material is selected from polyethylene films, polypropylene films and polyurethane films, having a thickness of 5 to 10 »m. The coating resinous liquid is selected from, for example, Daicure OL Medium (Trademark of a acrylic prepolymer, made by Dainihon Ink) and applied in an amount of 2 to 20 g/m² to the liquid-nonpermeable sheet. The printed sheet is fed into the curing section at a feed speed of 30 to 300 m/min, whereby the printed sheet is cured for a time of 0.5 to 30 seconds.

The ultraviolet ray radiation is carried out preferably by using an ultraviolet ray lamp, for example, a high pressure mercury lamp or metal halide lamp which generates ultraviolet rays having a wave length of 400 nm or less.

The number of ultraviolet ray lamps necessary for completing the curing of the printed resinous liquid layer varies and depends on the type and amount of the printed resinous liquid layer, the speed at which the printed sheet is fed, and the type and output of the ultraviolet ray lamp.

The electron beam radiation is preferably carried out by using a curtain beam type or scanning type electron beam accelerator, which is relatively cheap and capable of generating a large output. Preferably, the accelerating voltage is from 100 to 300 kV, at an absorption of 0.1 Mrad to 6 Mrad, more preferably 0.2 to 4.0 Mrad.

Usually, the resultant reinforcing resinous layer has a weight of 2 to 20 g/m², a width of 150 to 300 mm and an area of 75 to 300 cm².

When the weight is less than 2 g/m², the resultant reinforcing resinous layer sometimes exhibits an unsatisfactory reinforcing effect, and if the weight is more than 20 g/m², the irradiation of the ultraviolet rays or electron beam must be applied at an undesirably large intensity, to thoroughly cure the printed coating liquid layer, and thus the cost of the reinforcing resinous layer becomes undesirably high.

Referring to Figs. 1 and 3, the fastening tape member 7 preferably comprises a base member 7b and an adhesive tape member 7a extending outward from the base member 7a. The base member 7b is usually fixed on the back surface and/or the front surface of the diaper. Before a practical use of the diaper, the adhesive tape member 7a is usually folded and detachably adhered to the base member 7b.

The adhesive tape member has an adhesive layer composed of an adhesive (sticky) resinous material, for example, a mixture of natural rubber with rosin, a mixture of a styrene-butadiene rubber with a terpen resin, an ethylacrylate-vinyl acetate copolymer or a butylacrylate-methyl methacrylate copolymer.

The surface of the reinforcing resinous layer may be coated with a releasing agent comprising at least one member selected from silicone compounds, polyacrylic-compounds having a long alkyl group chain and polyvinyl alcohol compounds having a long alkyl group chain, to control the peeling strength of the adhesive layer of the adhesive tape member from the reinforcing resinous layer to the above-mentioned level. The releasing agent is mixed into the reinforcing resinous layer.

The improved reinforcing resinous layer of the present invention can be easily formed from the ultraviolet ray or electron beam-curable prepolymeric material at a low cost and high productivity, thoroughly reinforces the longitudinal end portion, preferably the front portion, of the diaper, and can be repeatedly fastened to the fastening tape members.

## Claims

1. A disposable diaper comprising:
(a) a liquid-nonpermeable back sheet (1) which forms an outside surface of the diaper;
(b) a liquid-absorptive core stratum (2) superimposed on a lateral middle portion of an inside surface of said back sheet (1);
(c) a liquid-permeable front sheet (3) covering at least an upper surface (2a) and both lateral side surfaces (2b) of said liquid absorptive core stratum; and
(d) a fastening tape system comprising
(i) at least one reinforcing resinous layer (6) coated on at least corner portions (5a) of a longitudinal end portion of an outside surface of the back sheet (1), and
(ii) a pair of fastening tape members (7) each having an adhesive tape member (7a) extending outward from a corner portion (4a) of the longitudinal opposite end portion of the outside surface of the back sheet (1) and having an adhesive layer formed on the inside surface thereof,
characterized in that the reinforcing resinous layer (6) comprises a mixture of a product of a synthetic polymeric material cured by an irradiation of ultraviolet rays or an electron beam, and a releasing agent, and whereby the adhesive tape members (7a) are able to be detachably adhered to a corresponding portion of the reinforcing resinous layer (6) at a peeling strength of 250 to 1000 g/25 mm, as determined in accordance with JIS Z 0237.

2. The disposable diaper as claimed in claim 1, wherein the reinforcing resinous layer (6) extends from one corner portion to the other corner portion of the longitudinal end portion of the outside surface of the back sheet (1).

3. The disposable diaper as claimed in claim 1, wherein the reinforcing resinous layer (6) is composed of a pair of reinforcing resinous layer segments (6a, 6a') spaced from each other and located on corner portions of the longitudinal end portion of the outside surface of the back sheet (1).

4. The disposable diaper as claimed in claim 1, 2 or 3, wherein the cured synthetic polymeric material consists of a prepolymeric material being cured by the irradiation of ultraviolet rays or an electron beam, a cross-bonding or diluting monomeric agent, and a polymerization-initiating agent.

5. The disposable diaper as claimed in claim 4, wherein the prepolymeric material comprises at least one member selected from the group consisting of:
(1) acrylate compounds of aliphatic, cycloaliphatic and aromatic mono- to hexa-hydric alcohols and of polyalkylene glycols,
(2) acrylate compounds consisting of addition reaction products of aliphatic, cycloaliphatic and aromatic mono- to hexa-hydric alcohols with alkylene oxides,
(3) polyacryloylalkyl phosphoric acid esters,
(4) reaction products of carboxylic acids with polyols and acrylic acid,
(5) reaction products of isocyanate compounds with polyols and acrylic acid,
(6) reaction products of epoxy group-containing compounds with acrylic acid, and
(7) reaction products of epoxy group-containing compounds with polyols and acrylic acid.

6. A process for producing a disposable diaper, comprising the steps of:
(a) printing a coating of resinous liquid (13) comprising a mixture of a synthetic polymeric material with a releasing agent, on at least corner portions (5a) of a back surface of a longitudinal end portion of a liquid-nonpermeable sheet (1, 11) to form at least one predetermined pattern and area, the synthetic polymeric material comprising a pre-polymeric material capable of being cured by the irradiation of ultraviolet rays or an electron beam, a cross-bonding or diluting monomeric agent, and a polymerization-initiating agent;
(b) curing the coating of resinous liquid by the irradiation of ultraviolet rays or electron beam to provide at least one reinforcing resinous layer (6);
(c) subjecting the liquid-nonpermeable sheet (1, 11) with the resultant reinforcing resinous layer (6) to a diaper-forming procedure in which the liquid-nonpermeable sheet (1, 11) is associated with a liquid-adsorptive core material (2, 18) and a liquid-permeable sheet material (3, 19) to form a diaper; and
(d) attaching a pair of adhesive tape members (7a) to the corner positions (4a) of the back surface of the longitudinal opposite end portion of the liquid-nonpermeable sheet (1, 11) through adhesive layers formed on the back surface corner portions of the diaper,
whereby the adhesive tape members can be detachably adhered to corresponding portions of the resultant reinforcing resinous layers at a peeling strength of 250 to 1000 g/25 mm, determined in accordance with Japanese Industrial Standard Z 0237.

7. The process as claimed in claim 6, wherein the release agent comprises at least one member selected from silicone compounds, polyacrylic compound having a long alkyl group chain, and polyvinyl alcohol compounds having a long alkyl group chain.

## Patentansprüche

1. Wegwerfwindel mit:
(a) einem flüssigkeitsundurchlässigen Rückblatt (1), das eine Außenfläche der Windel bildet;
(b) einer flüssigkeitsabsorbierenden Kernschicht (2), die auf einem seitlichen mittleren Abschnitt einer Innenfläche des Rückblatts (1) aufgebracht ist;
(c) einem flüssigkeitsdurchlässigen Frontblatt (3), das zumindest eine obere Oberfläche (2a) und beide seitlichen Seitenflächen (2b) der flüssigkeitsabsorbierenden Kernschicht abdeckt; und
(d) einem Befestigungsbandsystem mit
(i) zumindest einer verstärkenden Harzschicht (6), die mindestens auf Eckenbereichen (5a) eines längsverlaufenden Endbereichs einer Außenoberfläche des Rückblatts (1) aufgebracht ist, und
(ii) einem Paar von Befestigungsbandelementen (7), die jeweils ein Klebebandelement (7a) besitzen, das sich von einem Eckbereich (4a) des in Längsrichtung verlaufenden, entgegengesetzten Endbereichs der Außenoberfläche des Rückblatts (1) nach außen erstreckt und eine auf seiner Innenfläche gebildete Klebschicht aufweist,
dadurch gekennzeichnet, daß die verstärkende Harzschicht (6) eine Mischung aus einem Produkt aus synthetischem Polymermaterial, das durch eine Bestrahlung mittels ultravioletter Strahlen oder eines Elektronenstrahls gehärtet ist, und einem Trennmittel aufweist, wodurch die Klebebandelemente (7a) abnehmbar an einem entsprechenden Abschnitt der verstärkenden Harzschicht (6) mit einer Abziehstärke von 250 bis 1000 g/25 mm, die in Übereinstimmung mit JIS Z 0237 bestimmt ist, abziehbar sind.

2. Wegwerfwindel nach Anspruch 1, wobei sich die verstärkende Harzschicht (6) von einem Eckbereich zu dem anderen Eckbereich des in Längsrichtung verlaufenden Endbereichs der äußeren Oberfläche des Rückblatts (1) erstreckt.

3. Wegwerfwindel nach Anspruch 1, wobei die verstärkende Harzschicht (6) aus einem Paar verstärkender Harzschichtsegmente (6a, 6a') besteht, die voneinander beabstandet und an Eckbereichen des in Längsrichtung verlaufenden Endbereichs der Außenoberfläche des Rückblatts (1) angeordnet sind.

4. Wegwerfwindel nach Anspruch 1, 2 oder 3, wobei das gehärtete synthetische Polymermaterial aus einem Vorpolymerisatmaterial, das durch die Bestrahlung mittels ultravioletter Strahlen oder Elektronenstrahl gehärtet ist, einem querverbindenden oder verdünnenden Monomeragens und einem polymerisationseinleitenden Agens besteht.

5. Wegwerfwindel nach Anspruch 4, wobei das Vorpolymerisatmaterial zumindest ein Element enthält, das aus der Gruppe ausgewählt ist, die aus
(1) Acrylatverbindungen aus aliphatischen, cycloaliphatischen und aromatischen ein- bis sechswertigen Alkoholen und Polyalkylenglykolen,
(2) Acrylatverbindungen, die aus Additionsreaktionsprodukten von aliphatischen, cycloaliphatischen und aromatischen ein- bis sechswertigen Alkoholen mit Alkylenoxiden bzw. Alkoholethern bestehen,
(3) Polyacryloylalkylphosphorsäureestern,
(4) Reaktionsprodukten von Carboxylsäuren mit Polyolen und Acrylsäure,
(5) Reaktionsprodukten von Isocyanatverbindungen mit Polyolen und Acrylsäure,
(6) Reaktionsprodukten von Epoxidgruppen-enthaltenden Verbindungen mit Acrylsäure, und
(7) Reaktionsprodukten von Epoxidgruppen-enthaltenden Verbindungen mit Polyolen und Acrylsäure besteht.

6. Verfahren zum Herstellen einer Wegwerfwindel, mit den Schritten:
(a) Aufdrucken einer Beschichtung aus Harzflüssigkeit (13), die eine Mischung aus einem synthetischen Polymermaterial mit einem Trennmittel bzw. Trennmittel enthält, zumindest auf Eckbereichen (5a) einer Rückseite eines in Längsrichtung verlaufenden Endbereichs eines flüssigkeitsundurchlässigen Blatts (1, 11) zur Ausbildung zumindest eines vorbestimmten Musters und zumindest einer Fläche, wobei das synthetische Polymermaterial aus einem Vorpolymerisatmaterial, das durch die Bestrahlung mittels Ultraviolettstrahlen oder eines Elektronenstrahls härtbar ist, ein querverbindenden oder verdünnenden Monomeragens und einem polymerisationseinleitenden Agens besteht;
(b) Härten der Harzflüssigkeitsbeschichtung durch die Bestrahlung mit Ultraviolettstrahlen oder Elektronenstrahl zur Schaffung zumindest einer verstärkenden Harzschicht (6);
(c) Unterziehen des flüssigkeitsundurchlässigen Blatts (1, 11) mit der resultierenden verstärkenden Harzschicht (6) einem Windelherstellungsvorgang, bei dem das flüssigkeitsundurchlässige Blatt (1, 11) mit einem flüssigkeitsabsorbierenden Kernmaterial (2, 18) und einem flüssigkeitsdurchlässigen Blattmaterial (3, 19) zur Bildung einer Windel verbunden wird; und
(d) Anbringen eines Paars von Klebebandelementen (7a) auf die Eckbereiche (4a) der Rückseite des in Längsrichtung verlaufenden, entgegengesetzten Endbereichs des flüssigkeitsundurchlässigen Blatts (1, 11) mit Hilfe von Klebschichten, die auf den rückseitigen Eckbereichen der Windel gebildet sind,
wodurch die Klebebandelemente abnehmbar an entsprechenden Bereichen der resultierenden verstärkenden Harzschichten mit einer Abschälfestigkeit von 250 bis 1000 g/25 mm bestimmt in Übereinstimmung mit dem japanischen Industriestandard JIS Z 0237, angebracht werden können.

7. Verfahren nach Anspruch 6, bei dem das Trennmittel zumindest ein Element enthält, das aus Siliconverbindungen, Polyacrylverbindung mit einer langen Alkylgruppenkette und Polyvinylalkoholverbindungen mit einer langen Alkylgruppenkette ausgewählt ist.

## Revendications

1. Couche pour bébé à usage unique comprenant :
(a) une feuille inférieure imperméable à un liquide (1) qui forme la surface extérieure de la couche pour bébé ;
(b) une couche intermédiaire centrale absorbant un liquide (2), superposée sur une partie moyenne transversale de la surface intérieure de ladite feuille inférieure (1) ;
(c) une feuille supérieure perméable à un liquide (3) recouvrant au moins la surface supérieure (2a) et les deux surfaces des côtés latéraux (2b) de ladite couche intermédiaire centrale absorbant un liquide ; et
(d) un système de bande de fixation comprenant
(i) au moins une couche résineuse de renfort (6) appliquée sur au moins les parties de coin (5a) d'une extrémité longitudinale de la surface extérieure de la feuille inférieure (1) et
(ii) une paire d'éléments de bande de fixation (7) ayant chacun un élément de bande adhésive (7a) se prolongeant à l'extérieur à partir de la partie de coin (4a) de l'extrémité longitudinale opposée de la surface extérieure de la feuille inférieure (1) et ayant chacun une couche adhésive formée sur leur surface intérieure,
caractérisée en ce que la couche résineuse de renfort (6) comprend un mélange d'un produit d'une substance polymère synthétique vulcanisée par une irradiation par des rayons ultraviolets ou par un faisceau électronique, ainsi qu'un agent de décollement, les éléments de bande adhésive (7a) étant ainsi capables d'être fixés de manière amovible sur une partie correspondante de la couche résineuse de renfort (6) avec une résistance au décollement de 250 à 1 000 g/25 mm telle que déterminée selon JIS Z 0237.

2. Couche pour bébé à usage unique selon la revendication 1, dans laquelle la couche résineuse de renfort (6) s'étend d'une partie de coin à l'autre partie de coin de l'extrémité longitudinale de la surface extérieure de la feuille inférieure (1).

3. Couche pour bébé à usage unique selon la revendication 1, dans laquelle la couche résineuse de renfort (6) est constituée d'une paire de segments de couche résineuse de renfort (6a, 6a') espacés l'un de l'autre et situés sur les parties de coin de l'extrémité longitudinale de la surface extérieure de la feuille inférieure (1).

4. Couche pour bébé à usage unique selon la revendication 1, la revendication 2 ou la revendication 3, dans laquelle la substance polymère synthétique vulcanisée est constituée d'une substance prépolymère étant vulcanisée par l'irradiation par des rayons ultraviolets ou par un faisceau électronique, d'un agent monomère de réticulation ou de dilution, ainsi que d'un agent d'initiation de polymérisation.

5. Couche pour bébé à usage unique selon la revendication 4, dans laquelle la substance prépolymère comprend au moins un élément choisi dans le groupe constitué par les composés suivants :
(1) composés acrylates d'alcools monoatomiques à hexa-atomiques aliphatiques, cycloaliphatiques et aromatiques et composés acrylates de polyalkylèneglycols,
(2) composés acrylates constitués des produits de la réaction d'addition d'alcools monoatomiques à hexa-atomiques aliphatiques, cycloaliphatiques et aromatiques avec des oxydes d'alkylène,
(3) esters d'acides polyacryloylalkylphosphoriques,
(4) produits de la réaction d'acides carboxyliques avec des polyols et l'acide acrylique,
(5) produits de la réaction de composés isocyanates avec des polyols et l'acide acrylique,
(6) produits de la réaction de composés contenant un groupement époxy avec l'acide acrylique et
(7) produits de la réaction de composés contenant un groupement époxy avec des polyols et l'acide acrylique.

6. Procédé de production d'une couche pour bébé à usage unique comprenant les étapes consistant à :
(a) imprimer un revêtement de liquide résineux (13), comprenant un mélange d'une substance polymère synthétique et d'un agent de décollement, sur au moins les parties de coin (5a) de la surface inférieure d'une extrémité longitudinale d'une feuille imperméable à un liquide (1, 11) pour former au moins un motif et une zone prédéterminés, la substance polymère synthétique comprenant une substance prépolymère capable d'être vulcanisée par l'irradiation par des rayons ultraviolets ou par un faisceau électronique, un agent monomère de réticulation ou de dilution, ainsi qu'un agent d'initiation de polymérisation ;
(b) vulcaniser le revêtement de liquide résineux par l'irradiation par des rayons ultraviolets ou par un faisceau électronique pour fournir au moins une couche résineuse de renfort (6) ;
(c) soumettre la feuille imperméable à un liquide (1, 11) présentant la couche résineuse de renfort résultante (6) à une procédure de formation de couche pour bébé dans laquelle la feuille imperméable à un liquide (1, 11) est associée à un matériau central absorbant un liquide (2, 18) et à un matériau de feuille perméable à un liquide (3, 19) pour former une couche pour bébé ; et
(d) fixer une paire d'éléments de bande adhésive (7a) au niveau des coins (4a) de la surface inférieure de l'extrémité longitudinale opposée de la feuille imperméable à un liquide (1, 11) par des couches adhésives formées sur les parties de coin de la surface inférieure de la couche pour bébé,
pouvant ainsi coller de manière amovible les éléments de bande adhésive sur les parties correspondantes des couches résineuses de renfort résultantes avec une résistance au décollement de 250 à 1 000 g/25 mm, déterminée selon Japanese Industrial Standard Z 0237.

7. Procédé selon la revendication 6, dans lequel l'agent de décollement comprend au moins un élément choisi parmi des composés silicones, des composés polyacryliques ayant une longue chaîne à groupements alkyle et des composés poly(alcool vinylique) ayant une longue chaîne à groupements alkyle.
